(19)

**Europäisches Patentamt**
**European Patent Office**
**Office européen des brevets**

(11) **EP 4 629 899 B1**

(12) **EUROPEAN PATENT SPECIFICATION**

(45) Date of publication and mention
of the grant of the patent:
**18.03.2026 Bulletin 2026/12**

(21) Application number: **23821191.6**

(22) Date of filing: **06.12.2023**

(51) International Patent Classification (IPC):
*A61B 6/10* *(2006.01)*      *A61B 6/00* *(2024.01)*

(52) Cooperative Patent Classification (CPC):
**A61B 6/107; A61B 6/545; A61B 6/547;**
A61B 6/4441; A61B 6/542

(86) International application number:
**PCT/EP2023/084456**

(87) International publication number:
**WO 2024/121204 (13.06.2024 Gazette 2024/24)**

(54) **SYSTEM AND METHOD FOR REDUCING EXPOSURE TO X-RAY RADIATION BY POSITIONING X-RAY SOURCE**

SYSTEM UND VERFAHREN ZUR REDUZIERUNG DER RÖNTGENBELASTUNG DURCH POSITIONIERUNG EINER RÖNTGENQUELLE

SYSTÈME ET PROCÉDÉ DE RÉDUCTION D'EXPOSITION À UN RAYONNEMENT DE RAYONS X PAR POSITIONNEMENT D'UNE SOURCE DE RAYONS X

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **08.12.2022 US 202263431197 P**

(43) Date of publication of application:
**15.10.2025 Bulletin 2025/42**

(73) Proprietor: **Koninklijke Philips N.V.**
**5656 AG Eindhoven (NL)**

(72) Inventors:
• **SALEHI, Leili**
**5656 AG Eindhoven (NL)**
• **FOTOUHI, Javad**
**5656 AG Eindhoven (NL)**
• **SINHA, Ayushi**
**5656 AG Eindhoven (NL)**
• **ERKAMP, Ramon Quido**
**5656 AG Eindhoven (NL)**

• **PAI RAIKAR, Vipul Shrihari**
**5656 AG Eindhoven (NL)**
• **KYNE, Sean**
**5656 AG Eindhoven (NL)**

(74) Representative: **Philips Intellectual Property & Standards**
**High Tech Campus 52**
**5656 AG Eindhoven (NL)**

(56) References cited:
**US-A1- 2017 135 654      US-B2- 10 446 262**

• **RODAS NICOLAS LOY ET AL: "Pose optimization of a C-arm imaging device to reduce intraoperative radiation exposure of staff and patient during interventional procedures", 2017 IEEE INTERNATIONAL CONFERENCE ON ROBOTICS AND AUTOMATION (ICRA), IEEE, 29 May 2017 (2017-05-29), pages 4200 - 4207, XP033127225, DOI: 10.1109/ICRA.2017.7989483**

**Description**

**BACKGROUND**

Repeated exposure to high amounts of ionizing radiation may lead to health issues, such as erythema, hair loss, dermal atrophy, fibrosis, desquamation, dermal necrosis, cataracts, decrease in red blood cell production and infertility. For example, medical imaging that emits radiation (e.g., X-ray imaging) is needed to provide real-time and near real-time images during certain interventional procedures performed within a procedure room. Therefore, radiation exposure is a problem for many medical personnel, including physicians, interventionists, radiologists, and staff, as well as for patients, located within the procedure room during repeated procedures involving the emission of radiation. Interventionalists may also receive increased doses of radiation to their hands during several procedures. Even low amounts of radiation exposure may damage the genetic material in reproductive cells and increase chromosomal abnormalities. Radiation exposure may also alter DNA over time, as studies have shown increases in chromosomal abnormalities in medical personnel who are interventionalists, compared with those who are non-interventionalists.

Long term presence of the medical personnel procedure rooms using X-ray imaging systems, for example, may cause some health issues caused by ionizing radiation. The amount of the radiation dose emitted towards the medical personnel depends on C-arm orientation and location of the radiation source, patient size and position, and locations of medical personnel and patient relative to the C-arm/radiation source and the operating table. Protective shields and lead jackets may reduce the received doses of radiation, however they have limitations and drawbacks that contribute to the dissatisfaction of the medical personnel. Indeed, the limited size of the protective shields above the operating table and sometimes its improper position and orientation may increase the amount of radiation received by the medical personnel. In addition, protective lead jackets are cumbersome and heavy, and may cause musculoskeletal problems after long-term usage.

Consequently, it is critical to orient the C-arm of the medical imaging system such that medical personnel are not exposed to excessive radiation. The orientation of the C-arm and, therefore, the X-ray source also determine the quality of the view of the patient's anatomy that the X-ray imaging system acquires. A clear view of the target region of interest (ROI) and/or the path towards the target ROI plays a significant role in the success and failure of intervention procedures. Sometimes, obtaining such a view requires placing the X-ray radiation source in a position that exposes the medical personnel to large amounts of radiation due to orientation of the C-arm and/or inappropriate size, location, and orientation of the protective shields. Rodas Nicolas Loy et al: "Pose optimization of a C-arm imaging device to reduce intraoperative radiation exposure of staff and patient during interventional procedures", IEEE 29 May 2017, disclose an approach to optimize the imaging device's pose in order to reduce the exposure to radiation of both patient and staff, while preserving the visibility of the targeted anatomical structure in the acquired image.

Accordingly, there is a clinical need for reducing doses of radiation exposure to medical personnel by orienting the X-ray imaging system such that radiation exposure to medical personnel is minimized while still acquiring high quality views of the region of interest. This may include changing the X-ray source angulation to optimize viewing angle and radiation exposure reduction, rather than best viewing angle alone. Often, there are multiple optimal views for the same region of interest, and selecting a view that also reduces radiation exposure to medical personnel may not result in less optimal viewing angle. While in instances where multiple optimal views are not available this may result in slightly less optimal viewing angle, it significantly reduces radiation exposure to the medical personnel, as well as to the patient, without affecting the quality of care provided to the patient.

**SUMMARY**

The invention solves these problems by providing a system for reducing exposure to X-ray radiation as defined in claim 1 and, the corresponding method and computer program product as defined in claims 12 and 15, respectively. Particular embodiments are defined in the dependent claims.

**BRIEF DESCRIPTION OF THE DRAWINGS**

The example embodiments are best understood from the following detailed description when read with the accompanying drawing figures. It is emphasized that the various features are not necessarily drawn to scale. In fact, the dimensions may be arbitrarily increased or decreased for clarity of discussion. Wherever applicable and practical, like reference numerals refer to like elements.

FIG. 1 is a simplified block diagram of a system for reducing exposure to at least one clinician to X-ray radiation from an X-ray source in a procedure room, while providing adequate clinical information in images, according to a representative embodiment.

FIG. 2 provides schematic views of radiation patterns from different X-ray source positions and corresponding images of a patient, according to a representative embodiment.

FIG. 3 is a flow diagram of a method for reducing exposure to at least one clinician to X-ray radiation from an X-ray source of an X-ray imaging system, according to a representative embodiment.

FIG. 4 is a flow diagram of a method for training a system for reducing exposure to at least one clinician to X-ray radiation from an X-ray source in a procedure room, according to a representative embodiment.

## DETAILED DESCRIPTION

In the following detailed description, for purposes of explanation and not limitation, representative embodiments disclosing specific details are set forth in order to provide a thorough understanding of an embodiment according to the present teachings. Descriptions of known systems, devices, materials, methods of operation and methods of manufacture may be omitted so as to avoid obscuring the description of the representative embodiments. Nonetheless, systems, devices, materials, and methods that are within the purview of one of ordinary skilled in the art are within the scope of the present teachings and may be used in accordance with the representative embodiments. It is to be understood that the terminology used herein is for purposes of describing particular embodiments only, and is not intended to be limiting. The defined terms are in addition to the technical and scientific meanings of the defined terms as commonly understood and accepted in the technical field of the present teachings.

It will be understood that, although the terms first, second, third etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

The terminology used herein is for purposes of describing particular embodiments only, and is not intended to be limiting. As used in the specification and appended claims, the singular forms of terms "a," "an" and "the" are intended to include both singular and plural forms, unless the context clearly dictates otherwise. Additionally, the terms "comprises," and/or "comprising," and/or similar terms when used in this specification, specify the presence of stated features, elements, and/or components, but do not preclude the presence or addition of one or more other features, elements, components, and/or groups thereof. As used herein, the term "and/or" includes any and all combinations of one or more of the associated listed items.

Unless otherwise noted, when an element or component is said to be "connected to," "coupled to," or "adjacent to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" to another element or component, this encompasses only cases where the two elements or components are connected to each other without any intermediate or intervening elements or components.

In view of the foregoing, the present disclosure, through one or more of its various aspects, embodiments and/or specific features or sub-components, is thus intended to bring out one or more of the advantages as specifically noted below. For purposes of explanation and not limitation, example embodiments disclosing specific details are set forth in order to provide a thorough understanding of an embodiment according to the present teachings. However, other embodiments consistent with the present disclosure that depart from specific details disclosed herein remain within the scope of the appended claims. Moreover, descriptions of well-known apparatuses and methods may be omitted so as to not obscure the description of the example embodiments. Such methods and apparatuses are within the scope of the present disclosure.

Generally, the various embodiments provide deep-learning based algorithms for reducing exposure to one or more clinicians in a procedure room from radiation during operation of an X-ray imaging system, while maintaining acceptable image quality. The deep-learning algorithms use the viewing angle information from the X-ray source, which may be mounted on a C-arm, and position information from the one or more clinicians.

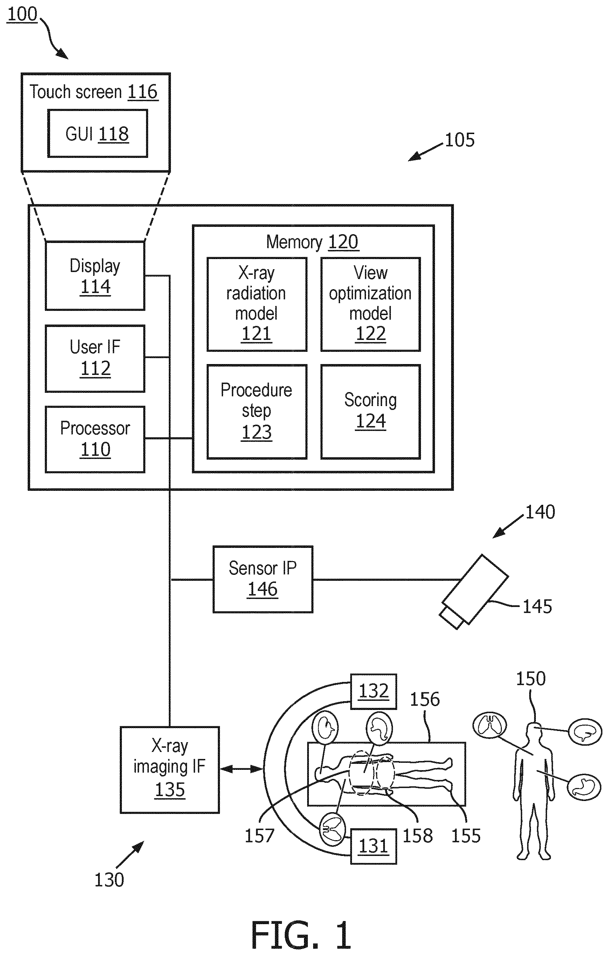

FIG. 1 is a simplified block diagram of a system for reducing exposure to at least one clinician to X-ray radiation from an X-ray source in a procedure room, while providing adequate clinical information in X-ray images according to a representative embodiment. The system reduces the radiation dose to the at least one clinician (e.g., a physician and procedure room staff) by changing the position of the X-ray source to optimize both the viewing angle for imaging purposes and the radiation exposure reduction. Often, there are multiple optimal views for the same region of interest, and selecting a view that also reduces radiation exposure to the at least one clinician may not result in less optimal viewing angle. While in instances where multiple optimal views are not available this may result in a slightly less than optimal viewing angle, it significantly reduces the radiation exposure to the at least one clinician without affecting the quality of care provided to the patient.

Referring to FIG. 1, system 100 includes a control unit 105 and an X-ray imaging system 130. The control unit 105 is configured to implement and/or manage the processes described herein. The control unit 105 includes one or more processors indicated by processor 110, one or more memories indicated by memory 120, a user interface (IF) 112, and a display 114. The memory 120 stores instructions executable by the processor 110. When executed, the instructions cause the processor 110 to implement one or more processes for reducing exposure to radiation of the at least one clinician, indicated by representative clinician 150, to the maximum extent, while still providing X-ray images that include adequate clinical information, as discussed below. "X-ray images that include clinical information" refers to X-ray images that are viewed from the appropriate angles so as to provide sufficiently clear and detailed views of the region of interest (ROI) to enable a medical professional to derive necessary medical information from the X-ray images. An example includes views of the aortic arch that show clear angulation of the outgoing carotid artery in order to enable the clinician 150 to navigate a guidewire device from the aortic arch into the carotid artery. Another example includes views of an intracranial artery that show the neck of the aneurysm allowing the clinician 150 to determine whether treatment is being successfully administered to the aneurysm without leakage into the blood vessel. As used herein, "clinician" refers to any personnel in the procedure room, such as an interventionalist, a cardiac surgeon, a radiology technician, an anesthesiologist, and a nurse, for example, each of whom may be exposed to radiation while performing a procedure on a patient 155. The procedure may be an interventional procedure, such as an interventional endovascular or endobronchial procedure (e.g., heart catheterization and transcatheter aortic valve replacement (TAVR)), for example. For purposes of illustration, the memory 120 is shown to include software modules, each of which includes the instructions corresponding to an associated capability of the control unit 105, as discussed below.

The processor 110 is representative of one or more processing devices, and may be implemented by a general purpose computer, a central processing unit (CPU), a computer processor, digital signal processor (DSP), a graphics processing unit (GPU), a microprocessor, a microcontroller, a state machine, programmable logic device, field programmable gate arrays (FPGAs), application specific integrated circuits (ASICs), or combinations thereof, using any combination of hardware, software, firmware, hard-wired logic circuits, or combinations thereof. Any processing device or processor herein may include multiple processors, parallel processors, or both. Multiple processors may be included in, or coupled to, a single device or multiple devices. The term "processor" as used herein encompasses an electronic component able to execute a program or machine executable instruction. A processor may also refer to a collection of processors within a single computer system or distributed among multiple computer systems, such as in a cloud-based or other multi-site application. Programs have software instructions performed by one or multiple processors that may be within the same computing device or which may be distributed across multiple computing devices.

The memory 120 may include main memory and/or static memory, where such memories may communicate with each other and the processor 110 via one or more buses. The memory 120 may be implemented by any number, type and combination of random access memory (RAM) and read-only memory (ROM), for example, and may store various types of information, such as software algorithms, artificial intelligence (AI) machine learning models, and computer programs, all of which are executable by the processor 110. The various types of ROM and RAM may include any number, type and combination of computer readable storage media, such as a disk drive, flash memory, an electrically programmable read-only memory (EPROM), an electrically erasable and programmable read only memory (EEPROM), registers, a hard disk, a removable disk, tape, compact disk read only memory (CD-ROM), digital versatile disk (DVD), floppy disk, Blu-ray disk, a universal serial bus (USB) drive, a solid state drive (SSD), or any other form of storage medium known in the art. The memory 120 is a tangible storage medium for storing data and executable software instructions, and is non-transitory during the time software instructions are stored therein. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. The memory 120 may store software instructions and/or computer readable code that enable performance of various functions. The memory 120 may be secure and/or encrypted, or unsecure and/or unencrypted.

The processor 110 and the memory 120 may include or have access to an AI engine or module, which may be implemented as software that provides artificial intelligence and machine learning algorithms, such as neural network modeling, described herein. The AI engine may reside in any of various components in addition to or other than the processor 110, such as the memory 120, an external server, and/or the cloud, for example. When the AI engine is implemented in a cloud, such as at a data center, for example, the AI engine may be connected to the processor 110 via the internet using one or more wired and/or wireless connection(s).

The user interface 112 is configured to provide information and data output by the processor 110 and/or the memory 120 to the user and/or to provide information and data input by the user to the processor 110 and/or the memory 120. That is, the user interface 112 enables the user to enter data and to control or manipulate aspects of the processes described herein, and to control or manipulate aspects of the X-ray imaging. The user interface 112 also enables the processor 110 to indicate the effects of the user's control or manipulation to the user.

All or a portion of the user interface 112 may be implemented by a graphical user interface (GUI), such as GUI 118 on a touch screen 116 of the display 114, for example. The user interface 112 includes push buttons operable (pushed) by the user to initiate various commands for manipulating the displayed image, making measurements and calculations, and the like during an imaging session (e.g., cone beam computed tomography "CBCT" or other X-ray examination) or at any point during an interventional procedure performed under X-ray guidance. The push buttons may be displayed by the GUI 118 on the touch screen 116, or may be physical buttons, for example. The user interface 112 may further include any other compatible interface devices, such as a mouse, a keyboard, a trackball, a joystick, microphone, a video camera, a touchpad, or voice or gesture recognition captured by a microphone or video camera, for example.

The display 114 may be any compatible monitor for displaying X-ray images, X-ray source positions and C-arm angles, and other information. For example, the display 114 may be a computer monitor, a liquid crystal display (LCD), an organic light emitting diode (OLED), a flat panel display, or a solid-state display. The display 114 includes the touch screen 116 and the GUI 118 to enable the user to interact with the displayed images and features, as discussed above.

The X-ray imaging system 130 includes an X-ray source 131 and an X-ray detector 132 connected in a fixed relationship to one another on a C-arm 133. The X-ray source 131 emits ionizing radiation, according to settings, such as dosage, frame rate, exposure time, and beam collimation, for example, that travels through a portion of the patient anatomy. The X-ray detector 132 receives the X-ray radiation that has traveled through patient anatomy and acquires X-ray images in response that enable visualization of the internal anatomy of the patient 155 on an operating table 156 in images. The images may include two-dimensional (2D) or three-dimensional (3D) images, such as X-ray images, fluoroscopy sequences, CBCT images, for example. When contrast is injected into the vasculature of the patient 155 during X-ray image acquisition, the visualization of the vascular anatomy of the patient 155 is enabled in images, such as digital subtraction angiography (DSA) images, 3D rotational angiography (3DRA) images, and computed tomography angiography (CTA) images, for example. The C-arm 133 is maneuverable for changing the location of the X-ray source 131 relative to the patient 155 to accommodate a variety of different viewing angles for the images. The X-ray imaging system 130 may be a fixed C-arm X-ray system mounted in the procedure room or a mobile C-arm X-ray system that is portable. Other configurations of the X-ray source 131 and X-ray detector 132 may also be envisioned such as in a C-less system where the X-ray source and the X-ray detector may move independently of each other.

More particularly, the X-ray source 131 is positioned relative to a region of interest (ROI) 157 of the patient 155 to obtain images of the patient's anatomy, for example, during an interventional procedure being performed by the clinician 150. The ROI 157 may be an operation site or access site for the interventional procedure, for example. An X-ray imaging interface 135 interfaces the X-ray imaging system 130 with the control unit 105 to convert X-ray image data to a format compatible with the processor 110 and/or to communicate X-ray imaging system information (e.g., encoded C-arm position) to the control unit 105. The control unit 105 sends control signals to the C-arm 133 for controlling movement of the C-arm 133 and placement of the X-ray detector 132 relative to the patient 155, and for controlling image acquisition including timing, frame rate, power and other imaging parameters, via the X-ray imaging interface 135. The control unit 105 also receives and processes the X-ray image data from the X-ray detector 132 in response to operation of the X-ray source 131. The clinician 150 (e.g., interventionalist or radiology technician) may control operations of the X-ray imaging system 130 through the user interface 112, although it is understood that control of the X-ray imaging system 130 may be partially or entirely performed through a separate control unit, without departing from the scope of the present teachings.

The position determination system 140 includes a position sensor 145 configured to provide position data indicating positions of the clinician 150, the patient 155 and objects in the procedure room (e.g., the operating table 156). The position sensor 145 may be a camera, such as RGB or an RGB-D camera, for example, that provides image data and depth information regarding the objects within its field of view, including the clinician 150, the patient 155 and various objects, for example. In an alternative embodiment, the position sensor 145 may include one or more position tracking devices, such as an electromagnetic (EM) detector or an optical sensor, for example. In this case, corresponding positioning devices, such as EM sensors or optical signal generators, respectively, would be attached to the people and objects in the field of view of the position sensor 145 for which position data is desired. For example, the clinician 150 may have EM sensors on a wearable badge, a wristband, garments, or the like.

The processor 110 receives the position data from the position sensor 145 via a sensor interface 146. When the position sensor 145 is a camera, for example, the position data include image data. The sensor interface 146 enables the position sensor 145 to send the position data to the processor 110 and to receive control commands from the processor 110 (e.g., adjusting imaging parameters, triggering image acquisitions). The processor 110 determines positions of the people and objects in three-dimensions by applying any compatible position determination algorithm to the position data, as would be apparent to one skilled in the art. For example, the processor 110 may determine the positions of the clinician 150, the patient 155, and the operating table 156 using the position data provided by the position sensor 145. The processor 110 may further determine the locations of the ROI 157 and any sensitive anatomical regions 158 of the patient 155 using the position data.

In the depicted embodiment, the memory 120 includes *inter alia* an X-ray radiation model module 121 for implementing an X-ray radiation model, a view optimization model module 122 for implementing a view optimization model, a procedure step module 123 for tracking steps of the specific procedure, and a scoring module 124 for scoring quality of images from different views of the X-ray source 131. The X-ray radiation model may comprise a mathematical model (also known as physics based model) for estimating X-ray radiation patterns, which include the impact of direct radiation from the X-ray source and scattered radiation from the direct radiation reflected from entities in the procedure room for any given angle of the C-arm 133. The X-ray radiation model may be analytically calculated (e.g., using Monte Carlo simulation) or estimated using a neural network, as would be apparent to one skilled in the art.

The X-ray radiation model of the X-ray radiation model module 121 inputs the position of at least the clinician 150, and optionally the positions of the patient 155, the operating table 156, and any other entities in the room that may reflect the X-ray radiation (e.g., walls, ceiling, tables), based on the position data provided by the position sensor 145; the positions (pose information) of the X-ray source 131 corresponding to viewing angles of the C-arm 133 for respective views of the ROI 157; and the X-ray related settings of the X-ray imaging system 130. Such settings may include dosage, frame rate, exposure time, and collimation of the X-ray radiation emitted by the X-ray source 131, for example. The X-ray radiation model may be a mathematical model, a physics-based simulation, or a data-driven solution for visualizing the respective radiation patterns.

The positions of the X-ray source 131 may be actual positions of the X-ray source 131 acquiring actual X-ray images of the patient 155, or may be simulated positions of the X-ray source 131 providing simulated X-ray images. In particular, the process of reducing radiation exposure may be performed using actual X-ray images acquired by the X-ray source 131 in different positions, or using simulated X-ray images derived from a simulated X-ray source in different positions with respect to a 3D or volumetric image of the ROI 157. The 3D image may be a pre-procedure or intra-procedure image acquired by the X-ray source 131 during a spin acquisition or by an alternative medical imaging system including a CT or an MRI system, or an image retrieved from a database, for example. When using a 3D image as input, the image of the ROI 157 is estimated from each of the different positions (views) of the X-ray source 131 by simulating X-rays traveling from the X-ray source 131 to the X-ray detector 132 through the patient anatomy as seen in the 3D image and computing the attenuation of the X-rays through the different tissue types, and the X-ray radiation model estimates the corresponding radiation pattern in the same manner as discussed above. The process of estimating the images, also known as digitally reconstructed radiographs (DRRs), from the different positions would be apparent to one skilled in the art. Generally, images and views refer to actual and simulated images and views from the X-ray source 131 in different positions.

Optimizing protection of the clinician 150 from X-ray radiation includes minimizing exposure to the clinician 150 to X-ray radiation that interacts with surfaces directly after being emitted from the X-ray source 131 and X-ray radiation reflected from surfaces of the entities present in the procedure room, such as the operating table 156 and other people and objects. After being emitted from the X-ray source 131, X-ray radiation that interacts directly with surfaces of entities may be referred to as "direct radiation," and radiation reflected from surfaces that subsequently interacts indirectly with surfaces of entities present in the procedure room may be referred to as "scattered radiation." The direct radiation and scattered radiation may be collectively referred to as "X-ray radiation," and the patterns or levels of exposure to surfaces of entities in the procedure room from the combined effects of direct and scattered radiation may be referred to as "X-ray radiation patterns" or simply "radiation patterns".

The X-ray radiation model outputs estimated radiation patterns corresponding to the positions of the X-ray source 131. Each of the radiation patterns includes the impact of the direct radiation and the scattered radiation and indicates how the radiation may spread when the X-ray source 131 is turned on at the particular settings. Each radiation pattern includes multiple levels of radiation, indicating levels of radiation exposure at different distances from the radiation source (the X-ray source 131). The direct radiation indicates radiation doses delivered to various entities in its path, including the clinician 150 and the patient 155, for example. The scattered radiation indicates how the X-ray beam interacts with (is reflected by) the various entities in its path, and allows computation of estimated radiation doses that may be delivered to entities outside the direct path of the X-ray beam emitted by the X-ray source 131.

In an embodiment, the X-ray radiation model may also generate a heatmap based on the radiation pattern. The heatmap is a visualization of the radiation pattern. The heatmap may indicate, using colors or shading, regions that are exposed to higher or lower amounts of X-ray radiation. The radiation pattern also may be visualized as scattered points (e.g., scatter plot or swarm plot), the densities of which correspond to the dosage levels of the X-ray radiation, or as contour lines defining corresponding areas having the same dosage.

The view optimization model of the view optimization model module 122 may comprise a neural network algorithm, such as an artificial neural network (ANN) algorithm, a convolutional neural network (CNN) algorithm, or a recurrent neural network (RNN) algorithm, for example. The view optimization model is configured to determine (predict) an optimal position of the X-ray source 131 from among the multiple positions of the X-ray source 131. The optimal position of the X-ray source 131 is the position in which the clinician 150 is exposed to the least amount of radiation while the X-ray imaging system 130 still provides an image of acceptable quality (i.e., that provides adequate clinical information). When there are multiple different positions of the X-ray source 131 that expose the clinician 150 to substantially the same least amount of radiation, the optimal position of the X-ray source 131 is the position of these multiple different positions that provides the best image quality. Likewise, when there are multiple different positions of the X-ray source 131 that provide substantially the same image quality, the optimal position of the X-ray source 131 is the position of these multiple different positions that exposes the clinician 150 to the least amount of radiation.

The view optimization model is configured to determine (predict) the optimal position of the X-ray source 131 by estimating exposure to the clinician 150 in the position indicated by the position data to X-ray radiation from the X-ray source 131 in positions corresponding to the various viewing angles that provide different views of the ROI 157, and identifying the optimal position of the X-ray source 131 that provides the best, acceptable image of the ROI 157 with the clinician 150 having minimum radiation exposure. The view optimization model inputs the position of the clinician 150, the multiple views provided by the X-ray source 131 from the different viewing angles, view quality scores corresponding to the multiple views, and the estimated radiation patterns corresponding to the positions of the X-ray source 131 at the different viewing angles. The view optimization model outputs the optimal position of the X-ray source 131 from the multiple positions that provides the highest view quality score from among view quality scores that provide adequate clinical information when viewing the ROI 157, while minimizing the radiation exposure to the clinician 150.

The position of the clinician 150 (as well as other personnel or objects whose position is to be taken into account by the view optimization model) is provided through the sensor interface 146 with reference to the coordinate system of the C-arm 133. If the position of the clinician 150 is in a different coordinate system (e.g., the coordinate system of the position sensor 145), the view optimization model may learn and compensate for the relation between that coordinate system and the coordinate system of the C-arm 133 during the training phase.

In an embodiment, the view optimization model may also input current projection geometry of the X-ray source 131. That is, the current position of the X-ray source 131 is also input into the view optimization model. The current position of the X-ray source 131 may be provided by the sensor interface 146 that is configured to additionally provide the position and orientation of the C-arm 133 and/or the X-ray source 131. In this case, the position of the clinician 150 and the X-ray source 131 are automatically represented within the same coordinate system. Alternatively, the current position of the X-ray source 131 may be obtained from the encoded C-arm 133 system. In this case, a relation between the coordinate system of the C-arm 133 and the coordinate system of the sensor interface 146 may be computed or learned by the view optimization model. The current projection geometry of the X-ray source 131 allows for a comparison between the current view and corresponding radiation pattern with the predicted view and corresponding radiation pattern to evaluate whether a view modification is necessary.

In an embodiment, the view optimization model also inputs information about the procedure step of the procedure being performed by the clinician, as determined by the procedure step module 123, discussed below. Using the procedure step, the processor 110 is able to associate views and/or view angles with the particular procedure step, and may also forecast views and/or view angles associated with future steps of the procedure. For example, each procedure step may require a different view angle, and the required view quality may vary in importance across procedure steps, such that different levels of radiation exposure may be tolerated for the different procedure steps. Therefore, the procedure step information also may be taken into account by the view optimization model when determining the optimal position of the X-ray source 131.

When the optimal position of the X-ray source 131 has been determined, the processor 110 visualizes (displays) the optimal position of the X-ray source 131 along with the position of the clinician 150 on the touch screen 116 of the GUI 118, for example. Other entities in the procedure room, such as other medical personnel, the patient 155, and the operating table 156, for example, may also be visualized on the touch screen 116, if desired. In an embodiment, the processor 110 may also display a visualization of the radiation pattern (e.g., a heatmap) corresponding to the optimal position of the X-ray source 131. This gives the clinician 150 a visual perspective on the extent of radiation exposure in his or her present position, and also enables the clinician 150 to determine whether to move to a different location, which would further minimize exposure to radiation. Alternatively, more favorable positions of the clinician 150 relative to the X-ray source 131 in its current position may also be calculated by the control unit 105, as discussed below. The control unit 105 may likewise include an augmented reality (AR) display, e.g., included in AR glasses worn by the user, which may similarly display a visualization of the radiation pattern and/or arrows indicating directions in which the clinician 150 may move to achieve a more optimal position with all other factors being equal.

In an embodiment, determination of the optimal position of the X-ray source 131 also takes into consideration locations of sensitive anatomical regions 158 of the patient 155, such as pelvic regions of reproductive age patients, for example. The view optimization model may then prioritize minimizing radiation exposure at this location, in addition to minimizing radiation exposure to the clinician 150.

When using supervised learning, any sensitive areas can be identified during training using bounding boxes or other region of interest indicators. For instance, areas to avoid exposing to radiation or to prioritize for imaging in computing the optimal position of the X-ray source 131 may be indicated by the clinician 150 or other user (e.g., by outlining areas to protect from radiation in red and areas to include in view in green on the user interface 112) in training data, allowing the view optimization model to optimize its weights during supervised training using loss functions that assign higher errors to X-ray source positions resulting in unacceptable levels of radiation exposure. This allows the view optimization model, for instance, to minimize or reduce radiation exposure in areas indicated by the user during inference or application of the view optimization model. For example, the user may interactively indicate on the touch screen 116 an area to protect, allowing the view optimization model's output of the optimal X-ray source position to be updated. Alternatively, the system may automatically infer which patients require additional anatomical regions to be protected from radiation exposure. For example, in an embodiment, the electronic health record (EHR) data of the patient may additionally be input into the view optimization model, allowing the view optimization model to access information, including patient age and sex, and to optimize its weights during supervised training such that X-ray source positions resulting in less radiation exposure to the identified pelvic areas of women of child-bearing age are prioritized, for example. By optimizing the weights of the view optimization model additionally based on the patient EHR data (including age, sex, and pregnancy status) during the training, the view optimization model learns from its parameters that features associated with identified regions of the patient anatomy must be protected. Therefore, the view optimization model will learn that for a pregnant woman or woman of child-bearing age, for example, the area around the abdomen and pelvic region must be protected, and will estimate X-ray source positions that prioritize minimizing radiation exposure to these regions for the relevant patients during inference, along with minimizing radiation exposure to the clinician 150.

In addition, the clinician 150 may enter via the user interface 112 and/or the GUI 118 acceptable levels of radiation based on his or her personal comfort level, so that the optimal position of the X-ray source 131 does not result in radiation exceeding the acceptable levels with clinician 150 in his or her present position. Alternatively or in addition, the control unit 105 may learn user preferences of the clinician 150 by keeping track of radiation levels previously accepted by the clinician 150. The user preferences may be input to the view optimization model to personalize the determination of the optimal position of the X-ray source 131 or may be learned automatically by the view optimization model by fine-tuning or updating its parameters based on positions of the X-ray source 131 accepted by the user during use of the system 100.

The view optimization model (e.g., neural network) is previously trained, using the processor 110 for example, before being implemented for an actual procedure. The training may include receiving historic data (actual and/or simulated) including previous positions of clinicians, patients, operating tables, and the like based on previous position data generated by the position sensor 145 during corresponding previous procedures. Previous position data of the C-arm 133 and acquired 3D data may be used to simulate previous positions of the X-ray source 131 and projection images that could be generated from the previous positions of the X-ray source 131. Alternatively, training data may include previous views and viewing angles of previous positions of the X-ray source 131 that provided the optimal view of the region of interest and/or the least exposure to radiation at the previous positions of the clinicians during the previous procedures. The training further includes estimating radiation patterns by the X-ray radiation model corresponding to the previous positions of the X-ray source 131. The previous positions of the clinicians, patients, the X-ray source, and operating tables, and the previous estimated radiation patterns from the X-ray radiation model may be retrieved from a database or other memory (e.g., memory 120), for example, accessible by the processor 110. The training results in the view optimization model outputting estimated positions of the X-ray source 131 that minimize the radiation exposures of the clinicians based on the previous position data and the estimated radiation patterns, while providing the highest quality images from among images that otherwise provide adequate clinical information when viewing the regions of interest.

The training includes various predetermined optimization criteria captured in corresponding loss functions. For example, one optimization criterion may be to minimize radiation exposure to the clinician 150 and/or other personnel standing closest to the X-ray source 131. Another optimization criterion may be to maximize view quality from among views that could be acquired by the C-arm. The optimization criterion may be a combination of several optimization criteria.

Different views of the ROI 157 may be required at different procedure steps. The current procedure step may be specified manually by the user or determined automatically. To determine the procedure step automatically, the procedure step module 123 receives the images acquired by the X-ray source 131 of the X-ray imaging system 130 in different positions, and outputs the procedure steps of the procedure associated with the images. The information about the procedure steps may be extracted from X-ray imaging system 130 via the X-ray imaging interface 135 by observing imaged anatomy or by tracking of interventional devices shown in the images. Alternatively, the information may be extracted from external audio and/or video capturing of interactions in the procedure room.

The procedure step module 123 may be implemented as a machine learning algorithm that, for instance, associates time elapsed in the procedure as well as positions of clinicians and tools visible in procedure room cameras with various procedure steps. For instance, if the radiology technician is positioned near and interacting with a contrast injector at the start of the procedure, this may indicate contrast injection immediately after a catheter has been inserted into the patient at the access site, and therefore the start of navigation towards the aortic arch. If the radiology technician is positioned near and interacting with the contrast injector later on in the procedure and aneurysm coils are placed near the patient, this may indicate the start of the treatment delivery phase.

The scoring module 124 includes a scoring model that receives images corresponding to views from the X-ray source 131 in different positions. These images may include actual images acquired by the X-ray source 131 in the different positions and/or simulated images derived from simulated X-ray source 131 in different simulated positions, as discussed above. The scoring model calculates and outputs a view quality score for each of the images from the different views. For example, the scoring module may calculate and output a view quality score for the quality of viewing a region of interest or other image feature in the images from the different views from the X-ray source. The view quality may be determined based on comparison of each image to an objective scale and/or to the other images. The scoring model of the scoring module 124 may evaluate the quality of the views of a region of interest such as overlap in the view of an aneurysm from other vasculature or other anatomy. The scoring model of the scoring module 124 may be implemented by a machine learning algorithm that is trained to evaluate the quality of the views of a region of interest based on historical procedure data. Also, the images input to the scoring model may be any of various types of X-ray images, such as DSA images, 3DRA images, or CTA images, for example.

The scoring model uses image features, such as vascular overlay or foreshortening, for example, to calculate an image quality score for each image. The scoring model may provide one or more view suggestions with corresponding DRRs, for example, computed from a 3D image, acquired intraoperatively or preoperatively, to the clinician 150, which may provide adequate clinical information for a current procedure step, while minimizing the radiation exposure to the clinician 150.

In an embodiment, a combined optimization is performed to find optimal view angle candidates from among the positions of the X-ray source 131 based on both the procedure step and minimal radiation exposure to the clinician 150 (and other personnel to the extent they are included in the view optimization model). In this case, during training of the view optimization model, a 3D image acquired at the start of or during the procedure may be used to evaluate 2D views via loss functions, which may be some combination of losses that evaluate the optimal view for the current procedure phase based on image features in DRRs and the optimal view for minimizing radiation exposure. An example of evaluating the optimal view for the current procedure phase may include determining view quality or view scores according to metrics appropriate for various procedure phases. For example, in the coil delivery phase of an aneurysm coiling procedure, an overlap between the aneurysm sac and the parent vessel may be determined in order to allow a clear view of the aneurysm neck. In the aortic arch crossing phase, an overlap between the ascending and descending aorta may be evaluated. An optimal view minimizes these overlaps. In another example, during the treatment planning phase of an aneurysm coiling procedure, the surface area of the aneurysm in 2D views may be evaluated. An optimal view according to this metric would maximize the surface area of the aneurysm so that accurate measurements of the aneurysm width can be made. Neural networks may be trained to learn image features in DRRs that are associated with optimal views according to these and other metrics, and to output view quality or scores that enable the selection of the best views. Neural networks can be trained by computing loss functions based on differences between predicted output data and expected output data using functions such as negative log-likelihood loss, mean squared error, Huber loss, or cross entropy loss. During training, the value of the loss function is typically minimized, and training is terminated when the value of the loss function satisfies a stopping criterion. The optimal view for minimizing the radiation exposure may be determined using mathematical models for radiation exposure, such as the X-ray radiation model of the X-ray radiation model module 121, described above. At inference, the trained view optimization model suggests view angles (and displays corresponding views) that are both optimal for the current procedure step and for minimal radiation exposure to clinician 150.

In an alternative embodiment, the optimal view for the current procedure step may be evaluated first by first using a 3D image to provide a set of candidate views of the ROI 157. The candidate views are then ranked in order of least to most radiation exposure to the clinician 150 in a particular position to find the view that minimizes the radiation exposure. This embodiment therefore first optimizes the best view for the procedure step alone, and then chooses from views optimized for the procedure step the view(s) that also minimize radiation exposure.

The system may use a data-driven approach to learn the ranking among the images, or alternatively may use an analytical solution to determine the ranking among different views. According to the data driven solution, the processor 110 (which may be referred to as a neural network controller) receives the position of the clinician 150, candidate poses of the C-arm 133 (and, optionally, simulated views from corresponding C-arm poses), and radiation patterns corresponding to the candidate C-arm poses as inputs. The processor 110 may also optionally receive the current projection geometry of the X-ray source 131.

The view optimization model of the view optimization model module 122, containing the learnable weights used for ranking the candidate poses and/or views, may be a neural network with convolutional and fully connected layers, as mentioned above. The view optimization model is used to learn relations between imaging content and view quality scores, and the fully connected layers may be used to fuse imaging data with non-imaging data, such as patient health information including patient age, weight, smoking history, and so forth. To combine different input data from various modalities, the view optimization model (neural network) may optionally use an ensemble of sub-networks (e.g., one network trained to process X-ray images and one network trained to process video data from the procedure room) or networks with a Siamese model architecture. The X-ray radiation model of the X-ray radiation model module 121 uses candidate C-arm poses and the positions of clinicians and other objects (and, optionally, synthetic imaging views from corresponding C-arm poses) to provide radiation patterns around the ROI 157 for each C-arm pose. The X-ray radiation model may use either physics-based simulation (e.g., Monte Carlo simulation) or data-driven solutions to estimate radiation patterns around the ROI 157. Information from X-ray characteristics, such as photon energy, collimation, scatters, beam-hardening, and noise, for example, may be used as inputs for both physics-based and data-driven solutions.

Training of the view optimization model and/or the X-ray radiation model may be performed either with full supervision using labeled data from procedures or from a simulation environment. In the simulation environment, different parameters, such as image settings and geometry of the X-ray imaging system 130 and positions of at least one clinician 150 may be varied, and the ranking of respective views with respect to radiation safety may be computed. The ranking of data is used to generate labeled data. During inference, the view optimization model inputs the current position(s) of the at least one clinician 150 in the procedure room and the output from the X-ray radiation model, and provides the ranking among the candidate views. The image with least total radiation exposure to the at least one clinician 150 will be ranked the highest.

According to the analytical solution, given all radiation pattern candidates, the processor 110 may alternatively select the appropriate view based on the maximum distance of the at least one clinician 150 to a level of radiation exposure in the radiation pattern. To this end, various distance functions may be employed to identify the view that corresponds to a level of radiation exposure in the radiation pattern with maximum distance to the at least one clinician 150. The distance functions may be provided in terms of continuous Euclidean distance or discrete classification distance.

Use of the continuous Euclidean distance selects the view of the X-ray source 131 that causes the total distance of the at least one clinician 150 to the centroid of the radiation pattern to be maximized, as provided by Equation (1):

$$\underset{j}{argmax} \; \Sigma \left| L_2 \left( d_i \left( R_j \right) \right) \right| \qquad \text{Equation (1)}$$

Referring to Equation (1), $d_i$ is a distance of each clinician i of the at least one clinician to the centroid of each of the radiation patterns, $L_2$ is a continuous loss function for $d_i$, and R is the radiation pattern for a given view where j is the number of candidate views.

Use of discrete classification distance may use binary cross entropy (BCE) to compute distance between the at least one clinician 150 and different zones within the radiation pattern, and similarly to select the view that maximizes the total distance between the at least one clinician 150 and corresponding radiation zones of the radiation pattern, as provided by Equation (2):

$$\underset{j}{argmax} \; \Sigma \left| BCE \left( \Delta_i \left( R_j \right) \right) \right| \qquad \text{Equation (2)}$$

Referring to Equation (2), $\Delta_i$ is a distance of each clinician i of the at least one clinician to a zone of each of the radiation patterns, BCE is the binary cross entropy function for $\Delta_i$, and $R_j$ is the radiation pattern for a given view where j is the number of candidate views. Both Equations (1) and (2) maximize the total distance between clinicians and a level of radiation exposure in the radiation pattern over the j candidate views. The j candidate views may be the set of optimal views for the current procedure step, and the view that maximizes the total distance between clinicians and a level of radiation exposure in the radiation pattern over the j candidate views is the optimal view for minimal radiation exposure.

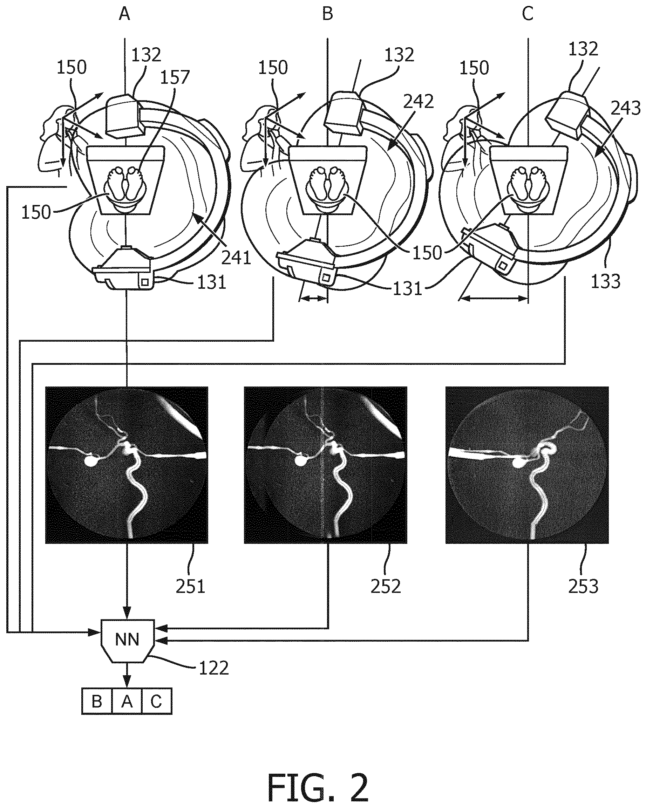

FIG. 2 provides schematic views of radiation patterns from different X-ray source positions and corresponding X-ray images of a patient, according to a representative embodiment.

Referring to FIG. 2, the view optimization model module 122 determines the order of preference with respect to three different views by the X-ray source 131 (and the C-arm 133) in three different candidate positions using continuous Euclidean distance or the discrete classification distance of the analytical solution. In particular, the X-ray source 131 is shown in three candidate positions for imaging the patient 155, indicated by first position A, second position B, and third position C. In the depicted example, the X-ray source 131 has a viewing angle of 0 degrees right anterior oblique (RAO) in the first position A, a viewing angle of about 10 degrees RAO in the second position B, and a viewing angle of about 25 degrees in the third position C. These three viewing angles generate the three most optimal views of the ROI 157 of the patient 155 for the current procedure step out of all feasible viewing angles of the C-arm. In all three positions of the X-ray source 131, the clinician 150 is shown at the same location.

FIG. 2 also shows illustrative radiation patterns respectively corresponding to the three positions of the X-ray source 131. In particular, first radiation pattern 241 corresponds to the first position A, second radiation pattern 242 corresponds to the second position B, and third radiation pattern 243 corresponds to the third position C. The radiation patterns may be determined using the trained X-ray radiation model from the X-ray radiation model module 121. The radiation patterns are shown as heatmaps with concentric areas indicated by different shadings corresponding to different levels of radiation exposure. Generally, the radiation exposure decreases moving from the innermost area to the outermost area of the heatmaps.

Likewise, FIG. 2 shows illustrative X-ray images displayed on the display 114 respectively corresponding to the three positions or viewing angles of the X-ray source 131. In particular, first image 251 corresponds to the first position A, second image 252 corresponds to the second position B, and third image 253 corresponds to the third position C. The images may be acquired through the X-ray imaging interface 135 or simulated from a 3D image of the ROI 157 of the patient 155. In the depicted example, the images are DSA images, although other types of X-ray based images may be incorporated without departing from the scope of the present teachings. The first, second and third images 251, 252 and 253 corresponding to positions A, B, and C are arranged in descending order of desirability with regard to view quality alone (highest image quality at first position A, lowest image quality at third position C).

As discussed above, the view optimization model of the view optimization model module 122 inputs the position of the clinician 150, the first, second and third images 251, 252 and 253 provided by the X-ray source 131 from the first, second and third positions A, B and C at the different viewing angles, view quality scores corresponding to the multiple images as provided by the scoring model, and the first, second and third radiation patterns 241, 242 and 243 corresponding to the first, second and third positions A, B and C of the X-ray source 131 at the different viewing angles. The view optimization model outputs the optimal position of the X-ray source 131, which in this example, is the second position B. This is consistent with the clinician 150 being completely outside a level of the second radiation pattern 242, combined with the fact that the first and second images 251 and 252 appear to be comparable in quality.

Further, in the depicted embodiment, the view optimization model ranks the first, second and third positions A, B and C in descending order of desirability with regard to low radiation exposure and good image quality. In this example, the positions of the X-ray source 131 are ranked in the order of the second position B, the first position A, and the third position C. Notably, the first position A and the third position C produce radiation patterns that result in similar exposure to the clinician 150 to X-ray radiation, as seen by comparing the first radiation pattern 241 to the third radiation pattern 243. However, the first position A is ranked ahead of the third position C with regard to preferability due to lower quality of the third image 253. In particular, the third image 253 shows an aneurism overlapping a vessel, which partially obstructs the view of the aneurysm, which is the ROI 157.

Another optimization criterion may be to protect the sensitive anatomical regions 158 of patients from direct radiation and/or scatter radiation. Similar to the discussion above, the sensitive anatomical regions 158 to protect may be annotated in the training data. In this case, the radiation exposure to the sensitive anatomical regions 158 from the X-ray source 131 with the C-arm 133 in different poses (different views) may be additionally minimized during optimization by penalizing C-arm 133 poses that result in high levels of radiation exposure to the sensitive anatomical regions 158. This may be done by modeling the X-ray beam generated from the X-ray source 131 given current X-ray settings (e.g., dosage, collimation) using the X-ray radiation model, and evaluating whether the resulting radiation pattern exposes the sensitive anatomical region to unacceptable levels of radiation.

Notably, the training of the view optimization model is described above with reference to the system 100, which is the same system used for subsequently performing the procedure for which the X-ray radiation model and the trained view optimization model are used. It is understood, however, that training data for training the view optimization model may be obtained from other systems, similarly configured to the system 100, without departing from the scope of the present teachings.

Also, in an embodiment, the training of the view optimization model may take place in a simulation environment, e.g., executable by the processor 110. The simulation environment generates data in the form of rendered scenes of the procedural environment where various parameters can be varied, such as X-ray image settings and geometry, and positions of clinicians and other entities, for example, in order to generate large amounts of simulated data. The optimal view and corresponding position of the X-ray source 131 may be modeled in the simulation based on radiation propagation from the X-ray radiation model and other physical characteristics. The simulated data may also be used in combination with real data, and may be used to evaluate the views and corresponding positions of the X-ray source 131 and to quantify how much reduction in radiation exposure is experienced at different clinician positions.

In another embodiment, the basis for selecting which view is to be the optimal view may be based on the current stage of the procedure, where each stage requires different criteria. For example, the criteria for selecting the optimal view for navigating an interventional instrument may differ from the criteria for selecting the optimal view for placing a stent, for example. The stage of the procedure may be determined automatically by the view optimization model using a current X-ray image acquired by the X-ray imaging system 130. For example, when the distal tip of an interventional instrument exceeds a predetermined threshold distance from a target within the patient 155, the view optimization model may determine that the procedure is in the navigation stage and identify corresponding criteria for what would constitute the optimal view. Likewise, when the distal tip of the interventional instrument is within another (shorter) predetermined threshold distance to the target, the view optimization model may determine that the procedure is in the stent placement stage and identify corresponding criteria. Alternatively or in addition, the stage of the procedure may be determined using external audio and/or video data of interactions in the procedure room, or may be defined by the clinician 150 via the user interface 112, for example. The view optimization model receives the radiation pattern from the X-ray radiation model, the positions of the clinician 150 and other personnel, and the candidate optimal views for the current stage of the procedure, and outputs optimal views from the candidate views that additionally minimize exposure to radiation.

In this embodiment, the user or interventionist manually selects a view to be their optimal view at the current stage of the procedure. The view optimization model receives the radiation patterns for all feasible C-arm angles from the X-ray radiation model, the positions of the clinician 150 and other personnel, and the user selected optimal view, and suggests one or more alternative views, where the alternative views are close to the user selected optimal view with regard to image quality, but can be achieved with less radiation exposure to the clinician 150 and/or the other personnel in the procedure room. Here the optimization criteria may additionally include minimizing a difference between the quality of the view selected by the user and the quality of the alternative views that minimize radiation exposure.

In another embodiment, after the optimal view has been identified/selected, the view optimization model determines small changes in the positions of the clinician 150 and/or other personnel in the procedure room to further minimize their radiation exposure, and the changes are provided to the clinician 150 and the other personnel as suggestions. The determination is based on the radiation pattern from the X-ray radiation model from the optimal view. For example, when the current position of the clinician 150 is at the periphery of a low dose range, as indicated by an isocontour in a radiation heatmap, a small movement away from the C-arm 133 may result in a significant change in radiation exposure. The view optimization model may consider distance from the operating table to set an upper limit on how much change in the position of the clinician 150 or other personnel may be suggested. For example, the position data may indicate that the clinician 150 (e.g., the interventionalist) is standing at the operating table, in which case the view optimization model would suggest little or no change in position since the clinician 150 would not have much flexibility. However, the position data may indicate that another person, e.g., a nurse, standing further away from the operation table, suggesting that that person may more easily be able to change their position to reduce their radiation exposure. The view optimization model may then determine a change of position for that person to the extent doing so would reduce radiation exposure.

In another embodiment, the view optimization model determines views to suggest to the clinician 150 additionally based on the previously established preferences of the clinician 150. For instance, the clinician 150 may consistently reject certain suggested views or define a range of C-arm angles of the C-arm 133 for the view optimization model to avoid based on personal comfort level. Alternatively, view optimization model may learn based on views that are typically selected by the clinician 150 or a user-defined range of preferred C-arm angles to suggest views that are preferred by the clinician 150. This may be implemented by weighting the loss function such that more desirable and/or more frequently used C-arm angles are preferred while less desirable and/or less used are suppressed, based on the preferences of the clinician 150. Less desirable C-arm angles may be suppressed by weighting the errors generated by less desirable C-arm angles such that they contribute more to the loss and, therefore, are not preferred by the neural network.

In another embodiment, the user interface 112 shows a set of suggested images and corresponding C-arm angles on the display 114 based on the current position of the clinician 150 and/or other personnel in the procedure room. The display 114 may also show the radiation pattern for the current C-arm angle, enabling the clinician 150 and the other personnel to adjust their positions of their own volition in order to receive lower amounts of radiation.

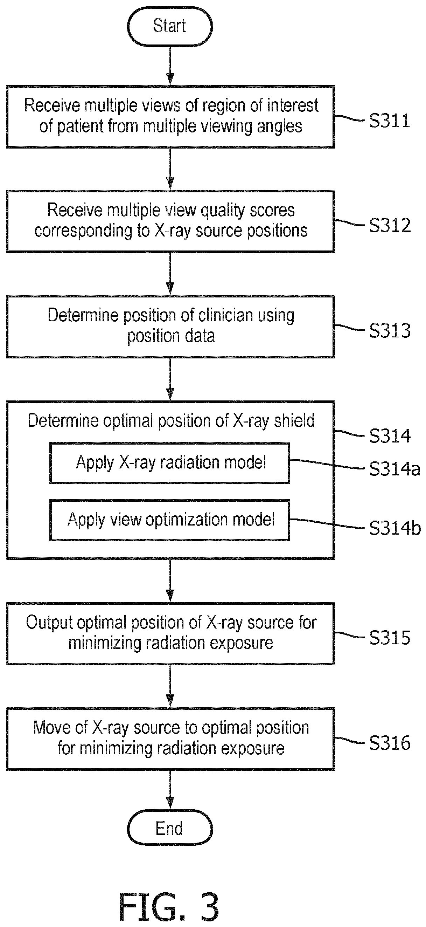

FIG. 3 is a flow diagram of a method for reducing exposure to at least one clinician to X-ray radiation from an X-ray source of an X-ray imaging system, according to a representative embodiment. The method depicted in FIG. 3 may be implemented by the processor 110 of the control unit 105, executing instructions stored in the memory 120, for example. Further, the steps of FIG. 3 are described in the context of one clinician for the sake of convenience, although it is understood that they apply equally to multiple clinicians to the extent more than one clinician is potentially exposed to X-ray radiation during the procedure. At least one clinician refers to one or more medical personal in the procedure room during operation of the X-ray imaging system, and may include an interventionalist, a cardiac surgeon, a radiology technician, an anesthesiologist, a nurse, a nurse, and the like.

Referring to FIG. 3, the method includes receiving multiple views of a region of interest of a patient (e.g., patient 155) from multiple viewing angles in block S311. The multiple viewing angles respectively correspond to multiple positions of the X-ray source (e.g., X-ray source 131) of the X-ray imaging system (e.g., X-ray imaging system 130). The X-ray imaging system is located in a procedure room, and is configured to image the region of interest of the patient during an interventional procedure, for example, which requires real time use of X-ray images. The X-ray source is configured to emit X-ray radiation (e.g., an X-ray beam) toward the patient 155 in accordance with X-ray settings, such as dosage, frame rate, exposure time, and collimation of the X-ray radiation. When the X-ray source is mounted on a C-arm of the X-ray imaging system, receiving the multiple views of the region of interest includes determining multiple angles of the C-arm corresponding to the multiple views.

The multiple views may be obtained from multiple X-ray images acquired by the X-ray source in different respective positions (i.e., different C-arm angles). Alternatively, the multiple views may be from a single 3D image, such as a pre-operative or intra-operative 3D image, for example, from which the multiple views are derived (e.g., simulated) for each of the different positions of the X-ray source, as discussed above.

In block S312, multiple view quality scores are determined, where the view quality scores respectively correspond to the positions of the X-ray source. The view quality scores are determined by a scoring model (e.g., from scoring module 124), which rates the quality of the images from each view against an objective quality standard and/or relative to the other images. In an embodiment, determining the view quality scores corresponding to the positions of the X-ray source may also include determining a stage of the procedure, and then determining at least one criterion for scoring the quality of the images based on the stage of the procedure. The stage of the procedure may be determined using a current X-ray image acquired by the X-ray imaging system or by receiving the stage of the procedure as defined by the clinician via a user interface (e.g., user interface 112).

In block S313, a position of the clinician in the procedure room is determined using position data received from a position sensor (e.g., position sensor 145). The position sensor may include a camera, such as RGB or an RGB-D camera, and/or a position tracking device, for example. Position data is provided for each clinician whose position is to be considered in minimizing exposure to radiation. Various embodiments may also include positions of other entities potentially affected by radiation exposure, such as the patient and anatomical regions of the patient. Position data may also be provided for other objects in the procedure room that may affect the radiation pattern in the room.

In block S314, an optimal position of the X-ray source is determined. The optimal position of the X-ray source is the position from which the X-ray source provides the highest view quality score from among multiple view quality scores that provide adequate clinical information when viewing the region of interest, while minimizing radiation exposure to the clinician to radiation originating from the X-ray source.

Determining the optimal position of the X-ray source includes applying an X-ray radiation model (e.g., provided by X-ray radiation model module 121) to the position of the clinician and multiple positions of the X-ray source to estimate multiple X-ray radiation patterns in block S314a, as discussed above. The X-ray radiation patterns include the impact of direct radiation from the X-ray source and scattered radiation reflected from entities within the procedure room. In an embodiment, the X-ray radiation model may be further applied to the X-ray settings of the X-ray source used to emit the X-ray radiation. Also, the X-ray radiation model may be configured to determine radiation patterns for the multiple positions of the X-ray source, where each radiation pattern may consist of concentric areas and/or isocontours corresponding to different levels of radiation exposure relative to the source of X-ray radiation. The radiation patterns may be determined using a mathematical model, a physics-based (e.g., Monte Carlo) simulation, or a data-driven solution.

Determining the optimal position further includes applying a view optimization model (e.g., provided by view optimization model module 122) to the position of the clinician, the multiple views corresponding to the multiple positions of the X-ray source, the quality scores corresponding to the multiple views, and the estimated radiation patterns corresponding to the multiple positions of the X-ray source to determine the optimal position of the X-ray source from the multiple positions in block S314b. The optimal position minimizes the clinician's exposure to the X-ray radiation. Applying the view optimization model to determine the optimal position of the X-ray source may include maximizing a distance of the clinician from a level of radiation exposure of multiple levels of radiation exposure over each of the radiation patterns using a distance function. The distance function may include a continuous function (e.g., Equation (1) above) that maximizes the distance between the clinician and a level of radiation exposure in each of the radiation patterns estimated for the multiple positions of the X-ray source. Alternatively, the distance function may include a discrete function (e.g., Equation (2) above) that maximizes the distance between the clinician and a zone of radiation exposure in each of the radiation patterns estimated for the multiple positions of the X-ray source.

In an embodiment, once the optimal position of the X-ray source is determined using the view optimization model, an adjustment to the position of the clinician may be determined relative to the optimal position of the X-ray source using a modified view optimization model, in order to further reduce the radiation exposure to the clinician. The modification to the view optimization model would optimize the position of the at least one clinician for a fixed C-arm pose instead of optimizing the C-arm pose for a fixed position of the at least one clinician. For example, a distance may be determined that a clinician can move without hindering their ability to perform a task in the procedure. The modified view optimization model may then determine a change in the position of the clinician within this distance such that the clinician is exposed to a lower level of radiation. The determined change or adjustment is output to the clinician, e.g., via the display, so that the clinician may move to the adjusted position, assuming doing so will not hinder the performance of the clinician during the procedure.

In another embodiment, the view optimization model is further applied to user preferences of the clinician to determine the optimal position of the X-ray source from the multiple positions. The user preferences may be determined automatically by the view optimization model by learning the preferences of each clinician during prior procedures, and then applying the preferences whenever the same clinician is identified at the start of a new procedure. Alternatively, the user preferences may be entered by the user, e.g., via the user interface, at the start of each procedure or during the procedure, and then applied by the view optimization model.

In block S315, the optimal position of the X-ray source is output to enable controlling the X-ray source to achieve the optimal position for acquiring X-ray images of the patient. In an embodiment, the output of the optimal position of the X-ray source may include visualizing the optimal position of the X-ray source on a display (e.g., display 114) and an indication of the radiation pattern (e.g., heatmap) of the X-ray originating from the X-ray source in the optimal position, as well as the current position of the clinician. This enables the clinician to see relative proximity to the radiation pattern. The clinician may readjust their position to place themselves in an area with even less radiation exposure. In an embodiment, the processing unit automatically calculates a new position with less radiation exposure, which information may be provided to the clinician as well, e.g., via the display, as discussed above. In an embodiment, the process further includes generating a digitally reconstructed radiograph (DRR) from a 3D image corresponding to the view of the region of interest from the optimal position of the X-ray source. The DRR may also be shown on the display, alone or along with the visualized radiation pattern.

In block S316, the X-ray source is moved from the current position to the optimal position output in block S315. The X-ray source may be moved automatically by the processor via the X-ray interface. The processor determines differences between the current position and the optimal position of the X-ray source, and issues commands to an X-ray interface to automatically drive the C-arm to move the X-ray source to the optimal position based on these differences. As discussed above, the X-ray interface may includes motors, actuators and/or other driving devices that operate in response to the commands from the processor. Alternatively, the X-ray source may be moved manually by a user (e.g., the clinician) by physically touching the X-ray source or by controlling the movement via the user interface, such as a GUI (e.g., GUI 118).

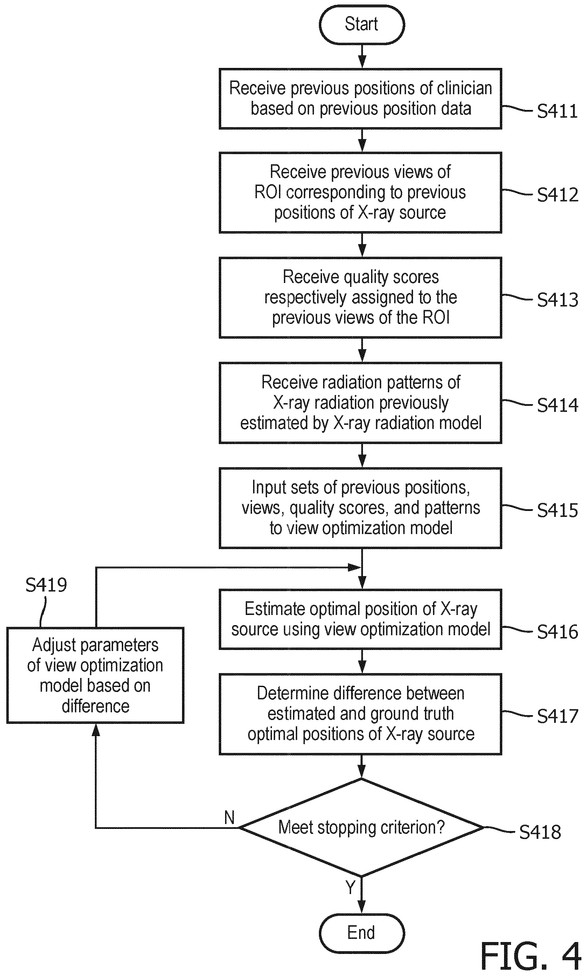

As discussed above, the view optimization model applied at block S314b is initially trained. FIG. 4 is a flow diagram of a method for training the view optimization model for reducing exposure to the at least one clinician to X-ray radiation from the X-ray source, while maintaining acceptable image quality, according to a representative embodiment. The method depicted in FIG. 4 may be implemented by the processor 110 of the control unit 105, executing instructions stored in the memory 120, for example. The training may be based on historic data that includes previous positions of clinicians, patients, and entities, previous positions of X-ray sources, and previously determined optimal positions of the X-ray source corresponding to positions of the at least one clinician, respectively. The historic data may be data from actual procedures previously performed using the same system, including the same control unit, X-ray imaging system and position sensor(s), or using different but similar systems. Alternatively, all or part of the historic data may be simulated, provided in a simulation environment.

Referring to FIG. 4, the method includes receiving previous positions of the clinicians based on previous position data generated by a position sensor (e.g., position sensor 145) during previous procedures on respective patients in block S411.

In block S412, previous views of the region of interest are received corresponding to positions of the X-ray source during the previous procedures. The previous views are from corresponding positions of the X-ray source/angles of the C-arm from which the previous views may have been acquired. Alternatively, previous views may be generated from simulated positions of the X-ray source/angles of the C-arm with respect to 3D images acquired during the previous procedures.

In block S413, previous view quality scores are received or calculated. In some embodiments, the quality scores have been respectively calculated for the previous views corresponding to the positions of the X-ray source/angles of the C-arm.

In block S414, radiation patterns of the X-ray radiation emitted by the X-ray source estimated by the X-ray radiation model are received. The radiation patterns are based on the previous position data of the clinicians providing corresponding sets of the previous position data, the positions of the X-ray source corresponding to views of the ROI, and X-ray related settings of the X-ray imaging system. The previous radiation patterns include the impact of direct radiation and scattered radiation estimated by the X-ray radiation model (e.g., X-ray radiation model module 121), where the direct radiation indicates X-ray radiation emitted from the X-ray source and scattered radiation indicates X-radiation reflected from entities within the procedure room.

In block S415, sets of the previous position data, the previous views of the ROIs provided by the X-ray source from different viewing angles, view quality scores corresponding to the previous views of the ROIs, and the previous estimated radiation patterns corresponding to the positions of the X-ray source at the different viewing angles are input to a view optimization model, which is the view optimization model being trained.

For each set of information, an optimal position (view) of the X-ray source that minimizes radiation exposure to the clinician at the position in the procedure room is estimated using the view optimization model in block S416, and the difference between the estimated optimal position and a ground truth optimal position of the X-ray source is determined in block S417. Estimating the optimal position of the X-ray source for the position of the clinician in block S416 may include applying loss functions, which include one or more of minimizing radiation exposure to the clinician who is standing closest to the X-ray source (when there are multiple clinicians), minimizing radiation exposure to sensitive anatomical regions the patients from X-ray radiation, maximizing a view quality of the view generated by the X-ray imaging system, or some combination thereof.

In block S418, it is determined whether a stopping criterion is met. For example, the stopping criterion may be when the difference between the estimated optimal position of the X-ray source and the ground truth optimal position of the X-ray source is less than a predetermined threshold. When the stopping criterion has not been met (block S418: No), the training of the view optimization model continues by adjusting the parameters of the view optimization model based on the difference between the estimated optimal position and a ground truth optimal position of the X-ray source in block S419, and repeating the estimating the optimal position of the X-ray source using the view optimization model with the updated parameters in block S416, and the comparing the estimated optimal position and the ground truth optimal position of the X-ray source in block S417. The parameters of the view optimization model are adjusted such that the difference between the estimated and the ground truth optimal positions is minimized. This is achieved using optimization techniques such as gradient descent. Various algorithms have been developed to implement these optimization techniques and their variants including but not limited to Stochastic Gradient Descent (SGD), batch gradient descent, mini-batch gradient descent, Gauss-Newton, Levenberg Marquardt, Momentum, Adam, and so forth. When the stopping criterion has been met (block S418: Yes), the training process ends, resulting in a trained view optimization model.

Although the present specification describes components and functions that may be implemented in particular embodiments with reference to particular standards and protocols, the disclosure is not limited to such standards and protocols. Such standards are periodically superseded by more efficient equivalents having essentially the same functions. Accordingly, replacement standards and protocols having the same or similar functions are considered equivalents thereof.

The illustrations of the embodiments described herein are intended to provide a general understanding of the structure of the various embodiments. The illustrations are not intended to serve as a complete description of all of the elements and features of the disclosure described herein. Many other embodiments may be apparent to those of skill in the art upon reviewing the disclosure. Other embodiments may be utilized and derived from the disclosure, such that structural and logical substitutions and changes may be made without departing from the scope of the disclosure. Additionally, the illustrations are merely representational and may not be drawn to scale. Certain proportions within the illustrations may be exaggerated, while other proportions may be minimized. Accordingly, the disclosure and the figures are to be regarded as illustrative rather than restrictive.

One or more embodiments of the disclosure may be referred to herein, individually and/or collectively, by the term "invention" merely for convenience and without intending to voluntarily limit the scope of this application to any particular invention or inventive concept. Moreover, although specific embodiments have been illustrated and described herein, it should be appreciated that any subsequent arrangement designed to achieve the same or similar purpose may be substituted for the specific embodiments shown. This disclosure is intended to cover any and all subsequent adaptations or variations of various embodiments. Combinations of the above embodiments, and other embodiments not specifically described herein, will be apparent to those of skill in the art upon reviewing the description.

In the foregoing Detailed Description, various features may be grouped together or described in a single embodiment for the purpose of streamlining the disclosure. This disclosure is not to be interpreted as reflecting an intention that the claimed embodiments require more features than are expressly recited in each claim. Rather, as the following claims reflect, inventive subject matter may be directed to less than all of the features of any of the disclosed embodiments. Thus, the following claims are incorporated into the Detailed Description, with each claim standing on its own as defining separately claimed subject matter.

The preceding description of the disclosed embodiments is provided to enable any person skilled in the art to practice the concepts described in the present disclosure.

**Claims**

1. A system (100) for reducing exposure to X-ray radiation, the system comprising:
   a processor (110) in communication with memory (120), the processor configured to:

   receive a plurality of views of a region of interest of a patient from a plurality of viewing angles corresponding to a plurality of positions of an X-ray source (131) of an X-ray imaging system (130) in a procedure room,
   calculate a plurality of view quality scores corresponding to the plurality of positions of the X-ray source, wherein the view quality scores rate the quality of the images of each view against an objective quality standard or relative to other images,
   determine a position of at least one clinician (150) in the procedure room based on position data received from at least one position sensor (145),
   estimate a plurality of radiation patterns of the X-ray radiation from the X-ray source corresponding to the plurality of positions of the X-ray source, and
   predict an optimal position of the X-ray source from the plurality of positions based on the position of the at least one clinician, the plurality of views, the plurality of view quality scores, and the plurality of estimated radiation patterns.

2. The system (100) of claim 1, wherein the X-ray source (131) is mounted on a C-arm (133) of the X-ray imaging system (130), and wherein the processor (110) is further configured to determine the plurality of views of the region of interest based on determination of a plurality of angles of the C-arm.

3. The system (100) of claim 1, wherein the optimal position of the X-ray source (131) provides a highest view quality score from among the view quality scores that provide adequate clinical information when viewing the region of interest, while minimizing radiation exposure to the at least one clinician.

4. The system (100) of claim 1, wherein, to calculate the plurality of view quality scores, the processor (110) is further configured to:

> determine a stage of the procedure (123) based on an X-ray image acquired by the X-ray imaging system (130) using at least one of external audio or video data of interactions within the procedure room, or by receiving the stage of the procedure as defined by the at least one clinician, and
> determine at least one criterion for scoring (124) view quality of the plurality of images based on the stage of the procedure.

5. The system (100) of claim 1, wherein the processor (110) is further configured to estimate the plurality of radiation patterns by applying an X-ray radiation model (121) that predicts the plurality of radiation patterns based on the position of the at least one clinician and each of the plurality of positions of the X-ray source (131), and wherein optionally the X-ray radiation model is configured to determine radiation patterns for the plurality of positions of the X-ray source using a mathematical model, a physics-based simulation, or a data-driven solution for visualizing the plurality of radiation patterns, respectively.

6. The system of claim 1,

> wherein the processor (110) is further configured to predict the optimal position of the X-ray source (131) by applying a view optimization model (122) that predicts the optimal position of the X-ray source based on the position of the at least one clinician (150), the plurality of views, the plurality of view quality scores, and the plurality of estimated radiation patterns, and
> wherein the view optimization model is configured to determine the optimal position of the X-ray source based on maximizing a distance of the at least one clinician from a level of radiation exposure of a plurality of levels of radiation exposure in each of the radiation patterns based on a distance function.

7. The system (100) of claim 1, wherein the processor (110) is further configured to:
determine an adjustment to the position of the at least one clinician (150) relative to the determined optimal position of the X-ray source (131) to further reduce the radiation exposure to the at least one clinician.

8. The system (100) of claim 6, wherein the processor (110) is further configured to predict the optimal position of the X-ray source (131) based on user preferences of the at least one clinician (150).

9. The system (100) of claim 1, wherein the processor (110) is further configured to:
display a volumetric heatmap indicating a radiation pattern of the X-ray source (131) in the optimal position that enables the at least one clinician to adjust position to further reduce the radiation exposure.

10. The system (100) of claim 1, wherein the processor (110) is further configured to determine the optimal position of the X-ray source (131) based on settings of the X-ray source, wherein the settings include dosage, frame rate, exposure time, and collimation of the radiation from the X-ray source.

11. The system (100) of claim 1, further comprising at least one of:

> the X-ray imaging system (130) comprising the X-ray source (131);
> the at least one sensor (145) configured to provide the position data indicating the position of the at least one clinician (150) in the procedure room; or
> at least one motor configured to move the X-ray source and/or a C-arm (133) attached to the X-ray source to position the X-ray source in the optimal position.

12. A method of reducing exposure to X-ray radiation, the method comprising:

> receiving a plurality of views of a region of interest of a patient from a plurality of viewing angles corresponding to a plurality of positions of an X-ray source (131) of an X-ray imaging system (130) in a procedure room;
> calculating a plurality of view quality scores corresponding to the plurality of positions of the X-ray source, wherein the view quality scores rate the quality of the images of each view against an objective quality standard or relative to other images;
> determining a position of at least one clinician (150) in the procedure room based on position data received from at least one position sensor (145);

estimate a plurality of radiation patterns of the X-ray radiation from the X-ray source corresponding to the plurality of positions of the X-ray source; and

predicting an optimal position of the X-ray source from the plurality of positions based on the position of the at least one clinician, the plurality of views, the plurality of view quality scores, and the plurality of estimated radiation patterns.

13. The method of claim 12, wherein the plurality of radiation patterns is estimated by applying an X-ray radiation model that predicts the plurality of radiation patterns based on the position of the at least one clinician (150) and each of the plurality of positions of the X-ray source (131).

14. The method of claim 13, wherein the optimal position of the X-ray source (131) is predicted by applying a view optimization model that predicts the optimal position of the X-ray source based on the position of the at least one clinician (150), the plurality of views, the plurality of view quality scores, and the plurality of estimated radiation patterns.

15. A computer program product comprising instructions for reducing exposure to X-ray radiation, the instructions, when executed by a processor (110), cause the processor to execute the method of claim 12.

**Patentansprüche**

1. System (100) zur Reduzierung der Aussetzung gegenüber Röntgenstrahlung, wobei das System umfasst:
einen Prozessor (110) in Kommunikation mit dem Speicher (120), wobei der Prozessor konfiguriert ist, um:

eine Vielzahl von Ansichten eines Bereichs von Interesse eines Patienten aus einer Vielzahl von Betrachtungswinkeln zu empfangen, die einer Vielzahl von Positionen einer Röntgenquelle (131) eines Röntgenbildgebungssystems (130) in einem Behandlungsraum entsprechen,
eine Vielzahl von Ansichtsqualitätsauswertungen, die der Vielzahl von Positionen der Röntgenquelle entsprechen, zu berechnen, wobei die Ansichtsqualitätsauswertungen die Qualität der Bilder jeder Ansicht gegenüber einem objektiven Qualitätsstandard oder in Bezug zu anderen Bildern bewerten,
eine Position von mindestens einem Kliniker (150) im Behandlungsraum basierend auf Positionsdaten zu bestimmen, die von mindestens einem Positionssensor (145) empfangen werden, bestimmen,
eine Vielzahl von Strahlungsmustern der Röntgenstrahlung von der Röntgenquelle zu schätzen, die der Vielzahl von Positionen der Röntgenquelle entsprechen, und
eine optimale Position der Röntgenquelle aus der Vielzahl von Positionen basierend auf der Position des mindestens einen Klinikers, der Vielzahl von Ansichten, der Vielzahl von Ansichtsqualitätsauswertungen und der Vielzahl von geschätzten Strahlungsmustern vorherzusagen.

2. System (100) nach Anspruch 1, wobei die Röntgenquelle (131) an einem C-Arm (133) des Röntgenbildgebungssystems (130) montiert ist und wobei der Prozessor (110) weiter konfiguriert ist, um die Vielzahl von Ansichten des Bereichs von Interesse basierend auf der Bestimmung einer Vielzahl von Winkeln des C-Armes zu bestimmen.

3. System (100) nach Anspruch 1, wobei die optimale Position der Röntgenquelle (131) eine höchste Ansichtsqualitätsauswertung aus den Ansichtsqualitätsauswertungen, die bei Betrachtung des Bereichs von Interesse ausreichende klinische Informationen bereitstellt, und gleichzeitig die Strahlungsaussetzung für den mindestens einen Kliniker minimiert.

4. System (100) nach Anspruch 1, wobei der Prozessor (110), um die Vielzahl von Ansichtsqualitätsauswertungen zu berechnen, weiter konfiguriert ist, um:

eine Behandlungsstufe (123) basierend auf einem vom Röntgensystem (130) erfassten Röntgenbildes unter Verwendung von mindestens einem von externen Audio- oder Videodaten von Interaktionen innerhalb des Behandlungsraumes oder durch Empfangen der Behandlungsstufe wie von dem mindestens einen Kliniker definiert zu bestimmen;
mindestens ein Kriterium zum Auswerten (124) der Ansichtsqualität der Vielzahl von Bildern basierend auf der Behandlungsstufe zu bestimmen.

5. System (100) nach Anspruch 1, wobei der Prozessor (110) weiter konfiguriert ist, um die Vielzahl von Strahlungsmustern durch Anwenden eines Röntgenstrahlungsmodell (121) zu schätzen, das die Vielzahl von Strahlungsmustern basierend auf der Position des mindestens einen Klinikers und jeder der Vielzahl von Positionen der Röntgenquelle (131) vorhersagt, und wobei das Röntgenstrahlungsmodell optional konfiguriert ist, um Strahlungsmuster für die Vielzahl von Positionen der Röntgenquelle unter Verwendung eines mathematischen Modells, einer physikalisch basierten Simulation oder einer datengetriebenen Lösung jeweils zur Visualisierung der Vielzahl von Strahlungsmustern zu bestimmen.

6. System nach Anspruch 1,

wobei der Prozessor (110) weiter konfiguriert ist, um die optimale Position der Röntgenquelle (131) durch Anwenden eines Ansichtsoptimierungsmodells (122) vorherzusagen, das die optimale Position der Röntgenquelle basierend auf der Position des mindestens einen Klinikers (150), der Vielzahl von Ansichten, der Vielzahl der Ansichtsqualitätsauswertungen und der Vielzahl der geschätzten Strahlungsmuster vorhersagt, und wobei das Ansichtsoptimierungsmodell konfiguriert ist, um die optimale Position der Röntgenquelle basierend auf Maximieren eines Abstands des mindestens einen Klinikers von einer Strahlungsaussetzungsstufe einer Vielzahl von Strahlungsaussetzungsstufen in jedem der Strahlungsmuster basierend auf einer Abstandsfunktion zu bestimmen.

7. System (100) nach Anspruch 1, wobei der Prozessor (110) weiter konfiguriert ist, um:
eine Anpassung der Position des mindestens einen Klinikers (150) in Bezug zur bestimmten optimalen Position der Röntgenquelle (131) zu bestimmen, um die Strahlungsaussetzung des mindestens einen Klinikers weiter zu reduzieren.

8. System (100) nach Anspruch 6, wobei der Prozessor (110) weiter konfiguriert ist, um die optimale Position der Röntgenquelle (131) basierend auf den Benutzerpräferenzen des mindestens einen Klinikers (150) vorherzusagen.

9. System (100) nach Anspruch 1, wobei der Prozessor (110) weiter konfiguriert ist, um:
eine volumetrische Heatmap, die ein Strahlungsmuster der Röntgenquelle (131) in der optimalen Position angibt, anzuzeigen, die es dem mindestens einen Kliniker ermöglicht, die Position anzupassen, um die Strahlungsaussetzung weiter zu reduzieren.

10. System (100) nach Anspruch 1, wobei der Prozessor (110) weiter konfiguriert ist, um die optimale Position der Röntgenquelle (131) basierend auf Einstellungen der Röntgenquelle zu bestimmen, wobei die Einstellungen die Dosis, die Einzelbildrate, die Belichtungszeit und die Kollimation der Strahlung von der Röntgenquelle beinhalten.

11. System (100) nach Anspruch 1, weiter mindestens eines umfassend von:

dem Röntgenbildgebungssystem (130), das die Röntgenquelle (131) umfasst;
mindestens einem Sensor (145), der konfiguriert ist, um Positionsdaten bereitzustellen, welche die Position des mindestens einen Klinikers (150) im Behandlungsraum angeben; oder
mindestens einem Motor, der konfiguriert ist, um die Röntgenquelle und/oder einen C-Arm (133), der an der Röntgenquelle befestigt ist, zu bewegen, um die Röntgenquelle in der optimalen Position zu positionieren.

12. Verfahren zur Reduzierung der Aussetzung gegenüber Röntgenstrahlung, wobei das Verfahren umfasst:

Empfangen einer Vielzahl von Ansichten eines Bereichs von Interesse eines Patienten aus einer Vielzahl von Betrachtungswinkeln, die einer Vielzahl von Positionen einer Röntgenquelle (131) eines Röntgenbildgebungssystems (130) in einem Behandlungsraum entsprechen;
Berechnen einer Vielzahl von Ansichtsqualitätsauswertungen, die der Vielzahl von Positionen der Röntgenquelle entsprechen, wobei die Ansichtsqualitätsauswertungen die Qualität der Bilder jeder Ansicht gegenüber einem objektiven Qualitätsstandard oder in Bezug zu anderen Bildern bewerten;
Bestimmen einer Position von mindestens einem Kliniker (150) im Behandlungsraum basierend auf Positionsdaten, die von mindestens einem Positionssensor (145) empfangen werden;
Schätzen einer Vielzahl von Strahlungsmustern der Röntgenstrahlung von der Röntgenquelle, die der Vielzahl von Positionen der Röntgenquelle entsprechen; und
Vorhersagen einer optimalen Position der Röntgenquelle aus der Vielzahl von Positionen basierend auf der Position des mindestens einen Klinikers, der Vielzahl von Ansichten, der Vielzahl von Ansichtsqualitätsauswertungen und der Vielzahl von geschätzten Strahlungsmustern.

**13.** Verfahren nach Anspruch 12, wobei die Vielzahl von Strahlungsmustern durch Anwendung eines Röntgenstrahlungsmodells geschätzt wird, das die Vielzahl von Strahlungsmustern basierend auf der Position des mindestens einen Klinikers (150) und jeder der Vielzahl der Positionen der Röntgenquelle (131) vorhersagt.

**14.** Verfahren nach Anspruch 13, wobei die optimale Position der Röntgenquelle (131) durch Anwenden eines Ansichtsoptimierungsmodells vorhergesagt wird, das die optimale Position der Röntgenquelle basierend auf der Position des mindestens einen Klinikers (150), der Vielzahl von Ansichten, der Vielzahl der Ansichtsqualitätsauswertungen und der Vielzahl der geschätzten Strahlungsmuster vorhersagt.

**15.** Computerprogrammprodukt, das Anweisungen zur Reduzierung der Aussetzung gegenüber Röntgenstrahlung umfasst, wobei die Anweisungen, wenn sie von einem Prozessor (110) ausgeführt werden, den Prozessor veranlassen, das Verfahren nach Anspruch 12 auszuführen.

**Revendications**

**1.** Système (100) de réduction de l'exposition au rayonnement de rayon X, le système comprenant :
un processeur (110) en communication avec une mémoire (120), le processeur étant configuré pour :

recevoir une pluralité de vues d'une région d'intérêt d'un patient à partir d'une pluralité d'angles de visualisation correspondant à une pluralité de positions d'une source de rayons X (131) d'un système d'imagerie à rayons X (130) dans une salle d'intervention,
calculer une pluralité de scores de qualité de vue correspondant à la pluralité de positions de la source de rayons X, dans lequel les scores de qualité de vue évaluent la qualité des images de chaque vue par rapport à une norme de qualité objective ou par rapport à d'autres images,
déterminer la position d'au moins un clinicien (150) dans la salle d'intervention en fonction des données de position reçues d'au moins un capteur de position (145),
estimer une pluralité de schémas du rayonnement de rayon X provenant de la source de rayons X correspondant à la pluralité de positions de la source de rayons X, et
prédire une position optimale de la source de rayons X à partir de la pluralité de positions en fonction de la position du au moins un clinicien, de la pluralité des vues, de la pluralité des scores de qualité de vue et de la pluralité des schémas de rayonnement estimés.

**2.** Système (100) selon la revendication 1, dans lequel la source de rayons X (131) est montée sur un bras en C (133) du système d'imagerie à rayons X (130), et dans lequel le processeur (110) est en outre configuré pour déterminer la pluralité de vues de la région d'intérêt en fonction de la détermination d'une pluralité d'angles du bras en C.

**3.** Système (100) selon la revendication 1, dans lequel la position optimale de la source de rayons X (131) fournit un score de qualité de vue le plus élevé parmi les scores de qualité de vue qui fournissent des informations cliniques adéquates lors de la visualisation de la région d'intérêt, tout en minimisant l'exposition au rayonnement du au moins un clinicien.

**4.** Système (100) selon la revendication 1, dans lequel pour calculer la pluralité de scores de qualité de vue, le processeur (110) est en outre configuré pour :

déterminer une étape de la procédure (123) sur la base d'une image radiographique acquise par le système d'imagerie à rayons X (130) en utilisant au moins une d'une donnée audio ou vidéo externe d'interactions au sein de la salle d'intervention, ou en recevant l'étape de la procédure telle que définie par le au moins un clinicien, et déterminer au moins un critère de notation (124) de la qualité de vue de la pluralité d'images en fonction de l'étape de la procédure.

**5.** Système (100) selon la revendication 1, dans lequel le processeur (110) est en outre configuré pour estimer la pluralité de schémas de rayonnement en appliquant un modèle de rayonnement de rayon X (121) qui prédit la pluralité de schémas de rayonnement en fonction de la position du au moins un clinicien et de chacune de la pluralité de positions de la source de rayons X (131), et dans lequel éventuellement, le modèle de rayonnement de rayon X est configuré pour déterminer les schémas de rayonnement pour la pluralité de positions de la source de rayons X en utilisant respectivement un modèle mathématique, une simulation basée sur la physique ou une solution basée sur les données pour visualiser la pluralité de schémas de rayonnement.

**6.** Système selon la revendication 1,

dans lequel le processeur (110) est en outre configuré pour prédire la position optimale de la source de rayons X (131) en appliquant un modèle d'optimisation de vue (122) qui prédit la position optimale de la source de rayons X en fonction de la position du au moins un clinicien (150), de la pluralité des vues, de la pluralité des scores de qualité de vue et de la pluralité des schémas de rayonnement estimés, et
dans lequel le modèle d'optimisation de vue est configuré pour déterminer la position optimale de la source de rayons X sur la base de la maximisation d'une distance du au moins un clinicien par rapport à un niveau d'exposition au rayonnement parmi une pluralité de niveaux d'exposition au rayonnement dans chacun des schémas de rayonnement, sur la base d'une fonction de distance.

**7.** Système (100) selon la revendication 1, dans lequel le processeur (110) est en outre configuré pour :
déterminer un ajustement de la position du au moins un clinicien (150) par rapport à la position optimale déterminée de la source de rayons X (131) afin de réduire davantage l'exposition au rayonnement du au moins un clinicien.

**8.** Système (100) de la revendication 6, dans lequel le processeur (110) est en outre configuré pour prédire la position optimale de la source de rayons X (131) en fonction des préférences utilisateur du au moins un clinicien (150).

**9.** Système (100) selon la revendication 1, dans lequel le processeur (110) est en outre configuré pour :
afficher une carte thermique volumétrique indiquant un schéma de rayonnement de la source de rayons X (131) dans la position optimale qui permet à le au moins un clinicien d'ajuster la position pour réduire davantage l'exposition au rayonnement.

**10.** Système (100) selon la revendication 1, dans lequel le processeur (110) est en outre configuré pour déterminer la position optimale de la source de rayons X (131) en fonction des paramètres de la source de rayons X, dans lequel les paramètres incluent le dosage, la fréquence d'images, le temps d'exposition et la collimation du rayonnement de la source de rayons X.

**11.** Système (100) selon la revendication 1, comprenant en outre au moins un des éléments suivants :

le système d'imagerie à rayons X (130) comprenant la source de rayons X (131) ;
le au moins un capteur (145) configuré pour fournir les données de position indiquant la position du au moins un clinicien (150) dans la salle d'intervention ; ou
au moins un moteur configuré pour déplacer la source de rayons X et/ou un bras en C (133) fixé à la source de rayons X pour positionner la source de rayons X dans la position optimale.

**12.** Procédé de réduction de l'exposition au rayonnement de rayon X, le procédé comprenant :

la réception d'une pluralité de vues d'une région d'intérêt d'un patient à partir d'une pluralité d'angles de visualisation correspondant à une pluralité de positions d'une source de rayons X (131) d'un système d'imagerie à rayons X (130) dans une salle d'intervention ;
le calcul d'une pluralité de scores de qualité de vue correspondant à la pluralité de positions de la source de rayons X, dans lequel les scores de qualité de vue évaluent la qualité des images de chaque vue par rapport à une norme de qualité objective ou par rapport à d'autres images ;
la détermination de la position d'au moins un clinicien (150) dans la salle d'intervention en fonction des données de position reçues d'au moins un capteur de position (145) ;
l'estimation d'une pluralité de schémas de rayonnement de rayon X provenant de la source de rayons X correspondant à la pluralité de positions de la source de rayons X ; et
la prédiction d'une position optimale de la source de rayons X à partir de la pluralité de positions en fonction de la position du au moins un clinicien, de la pluralité des vues, de la pluralité des scores de qualité de vue et de la pluralité des schémas de rayonnement estimés.

**13.** Procédé selon la revendication 12, dans lequel la pluralité des schémas de rayonnement est estimée en appliquant un modèle de rayonnement de rayon X qui prédit la pluralité des schémas de rayonnement en fonction de la position du au moins un clinicien (150) et de chacune des pluralités de positions de la source de rayons X (131).

**14.** Procédé selon la revendication 13, dans lequel la position optimale de la source de rayons X (131) est prédite en appliquant un modèle d'optimisation de vue qui prédit la position optimale de la source de rayons X en fonction de la position du au moins un clinicien (150), de la pluralité des vues, de la pluralité des scores de qualité de vue et de la pluralité des modèles de rayonnement estimés.

15. Produit de programme informatique comprenant des instructions pour réduire l'exposition au rayonnement de rayon X, les instructions, lorsqu'elles sont exécutées par un processeur (110), amènent le processeur à exécuter le procédé selon la revendication 12.

FIG. 1

FIG. 2

```
                    ┌─────────┐
                    │  Start  │
                    └─────────┘
                         │
                         ▼
      ┌──────────────────────────────────────┐
      │  Receive multiple views of region of  │
      │    interest                            │── S311
      │  of patient from multiple viewing angles│
      └──────────────────────────────────────┘
                         │
                         ▼
      ┌──────────────────────────────────────┐
      │   Receive multiple view quality scores │── S312
      │  corresponding to X-ray source positions│
      └──────────────────────────────────────┘
                         │
                         ▼
      ┌──────────────────────────────────────┐
      │   Determine position of clinician using│── S313
      │            position data               │
      └──────────────────────────────────────┘
                         │
                         ▼
      ┌──────────────────────────────────────┐
      │  Determine optimal position of X-ray shield│── S314
      │  ┌────────────────────────────────┐  │
      │  │   Apply X-ray radiation model  │  │── S314a
      │  └────────────────────────────────┘  │
      │  ┌────────────────────────────────┐  │
      │  │  Apply view optimization model │  │── S314b
      │  └────────────────────────────────┘  │
      └──────────────────────────────────────┘
                         │
                         ▼
      ┌──────────────────────────────────────┐
      │   Output optimal position of X-ray source for│── S315
      │       minimizing radiation exposure    │
      └──────────────────────────────────────┘
                         │
                         ▼
      ┌──────────────────────────────────────┐
      │   Move of X-ray source to optimal position│── S316
      │      for minimizing radiation exposure │
      └──────────────────────────────────────┘
                         │
                         ▼
                    ┌─────────┐
                    │   End   │
                    └─────────┘
```

# FIG. 3

```
                            ( Start )
                               │
                               ▼
        ┌─────────────────────────────────────────┐
        │ Receive previous positions of clinician  │──── S411
        │    based on previous position data       │
        └─────────────────────────────────────────┘
                               │
                               ▼
        ┌─────────────────────────────────────────┐
        │          Receive previous views of       │
        │    ROI corresponding to previous         │──── S412
        │         positions of X-ray source        │
        └─────────────────────────────────────────┘
                               │
                               ▼
        ┌─────────────────────────────────────────┐
        │          Receive quality scores          │
        │     respectively assigned to the         │──── S413
        │          previous views of the ROI       │
        └─────────────────────────────────────────┘
                               │
                               ▼
        ┌─────────────────────────────────────────┐
        │      Receive radiation patterns of       │
        │        X-ray radiation previously        │──── S414
        │    estimated by X-ray radiation model    │
        └─────────────────────────────────────────┘
                               │
                               ▼
        ┌─────────────────────────────────────────┐
        │   Input sets of previous positions,      │
        │   views, quality scores, and patterns    │──── S415
        │        to view optimization model        │
        └─────────────────────────────────────────┘
                               │
   S419                        ▼
   ┌──────────────────┐  ┌─────────────────────────────────────────┐
   │  Adjust parameters│  │   Estimate optimal position of X-ray    │──── S416
   │  of view optimization│  │  source using view optimization model │
   │  model based on  │  └─────────────────────────────────────────┘
   │    difference    │                 │
   └──────────────────┘                 ▼
            ▲         ┌─────────────────────────────────────────┐
            │         │    Determine difference between         │
            │         │      estimated and ground truth         │──── S417
            │         │   optimal positions of X-ray source     │
            │         └─────────────────────────────────────────┘
            │                           │
            │                           ▼
            │   N            ◇ Meet stopping criterion? ◇──── S418
            └───────────────┘              │
                                           Y
                                           ▼
                                        ( End )
```

## FIG. 4

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **RODAS NICOLAS LOY et al.** Pose optimization of a C-arm imaging device to reduce intraoperative radiation exposure of staff and patient during interventional procedures. IEEE, 29 May 2017